# EUROPEAN PATENT APPLICATION

(11) **EP 4 803 079 A1**
(43) Date of publication of application: **09.09.2026**
(21) Application number: 24885863.1
(22) Date of filing: 01.11.2024
(51) Int. Cl.: A61K 31/4045, A61K 31/42, A61K 31/4184, A61P 25/00, A61P 25/28

(54) **THERAPEUTIC AGENT FOR FRONTOTEMPORAL LOBAR DEGENERATION AND THERAPEUTIC COMPOSITION**

(30) Priority: 02.11.2023 JP 2023188497; 02.10.2024 JP 2024173803
(71) Applicant: K Pharma, Inc., Tokyo 106-0032 (JP)
(72) Inventor: KOKUBU Hiroshi, Fujisawa-shi, Kanagawa 251-8555 (JP)
(74) Representative: Müller, Christian Stefan Gerd
(86) International application number: PCT/JP2024/039021
(87) International publication number: WO 2025/095099

(57) **Abstract**

The present invention addresses the problem of providing: a therapeutic agent for frontotemporal lobar degeneration (FTLD) or a therapeutic composition for FTLD for which no therapeutic drug has been developed so far; and a method for treating FTLD. The inventors of the present invention have found that the frontal cortical neurons were differentiated from iPS cells derived from FTLD patients and then the pathology of FTLD in frontal cortical neurons was improved by administering to the frontal cortical neurons that reflect the pathology of FTLD with the therapeutic agent for a frontotemporal lobar degeneration (FTLD) comprising a compound represented by the following formula (1-1): 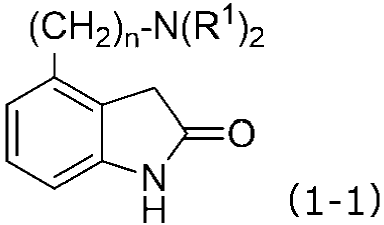 a compound represented by the following formula (2-1): 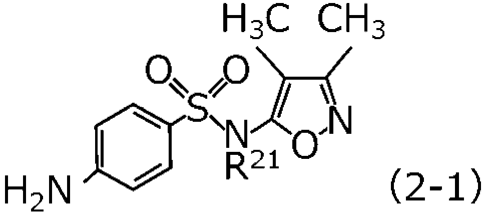 or a compound represented by the following formula (3-1): 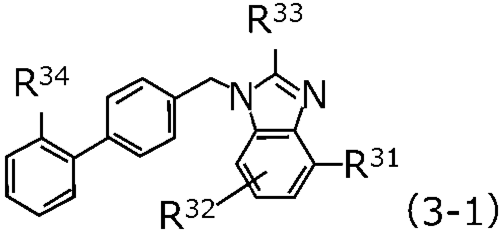 a pharmaceutically acceptable salt thereof, or a solvate thereof.

## Description

### Technical Field

The present invention relates to develop a therapeutic agent or composition for frontotemporal lobar degeneration (FTLD).

### Background Art

Frontotemporal lobar degeneration (FTLD) is a type of dementia characterized by degeneration of the frontal and temporal lobes, primarily manifesting symptoms such as behavioral and language disorders. It is designated as Designated Intractable Disease in Japan (Designated Intractable Disease 127), with an estimated 12,000 patients (see, Non-Patent Literature 1), 50,000-60,000 patients in the United States, and over 100,000 patients in Europe.

FTLD is broadly classified into three subtypes based on clinical symptoms, with the characteristics shown in the table below:
- Behavioral variant frontotemporal dementia (bvFTD)
- Semantic dementia (SD)
- Progressive non-fluent aphasia (PNFA).

**[Table 1]**

| Frontotemporal lobar degeneration | **English notation** | **prognosis** |
|---|---|---|
| Behavioral variant frontotemporal dementia | bvFTD | 7-9 years |
| Semantic dementia | SD | 10-12 years |
| Progressive non-fluent aphasia | PNFA | about 10 years |

FTLD is a pathologically and genetically diverse disease, and, depending on the involved protein, it can be classified into FTLD-tau (45%), in which TAU accumulation is observed; FTLD-TDP43 (45%), in which TDP-43 accumulation is observed; and FTLD-FUS (9%), in which FUS accumulation is observed. Furthermore, from a genetic perspective, the *GRN* gene, *MAPT* gene, *C9ORF72* gene, and *TARDBP* gene are known to be typical genes that generate mutations.

Frontotemporal dementia (FTD) is a comprehensive group of non-Alzheimer's degenerative dementia diseases primarily characterized by lesions in the motor cortex and some cases of which are known to be caused by TAU degeneration, TDP-43, and FUS. TDP-43 and FUS-related FTD are divided into those accompanied by movement disorders (amyotrophic lateral sclerosis) (FTLD-MND) and those not accompanied by movement disorders (FTLD-nonMND). Meanwhile, FTD caused by TAU degeneration are further divided into Pick's disease, characterized by 3R Tau degeneration, and diseases characterized by 4R TAU degeneration (corticobasal degeneration, progressive supranuclear palsy, and argyrophilic grain dementia).

In a diagnosis based on the pathology of the disease, bvFTD can be diagnosed if three or more of the following evaluation criteria A to F are met:
A. Behavioral Disinhibition: One or more of the following three symptoms must be present:
   1) Socially inappropriate behavior
   2) Loss of manners or decorum
   3) Impulsive, rash or careless actions
B. Inertia or apathy
C. Loss of sympathy or empathy: One or more of the following symptoms must be present:
   1) Diminished response to other people's needs and feelings
   2) Diminished social interest, interrelatedness or personal warmth
D. Perseverative or stereotyped behavior: One or more of the following symptoms must be present:
   1) Simple repetitive movements
   2) Compulsive or ritualistic behaviors
   3) Stereotypy of speech
E. Hyperorality and dietary changes: One or more of the following symptoms must be present
   1) Altered food preferences
   2) Binge eating, increased consumption of alcohol or cigarettes
   3) Oral exploration or consumption of inedible objects
F. Neuropsychological profile: Executive/generation deficits with relative sparing of memory and visuospatial functions

In the case of SD, at least three among the following four symptoms are present: A. Impaired object knowledge (especially low frequency/low familiarity), B. Surface dyslexia or dysgraphia, C. Spared repetition (fluent speech is observed), D. Spared speech production (grammar and spontaneous speech),

Currently, there is no established treatment aimed at curing FTLD (improvement of cognitive function), and symptomatic treatment is used to treat some of the symptoms. As such symptomatic treatment, for example, it has been reported that administering selective serotonin reuptake inhibitors (SSRIs), an antidepressant, is effective when behavioral disturbances are prominent, and it is also known that, although at the case level, antipsychotics and antiepileptic drugs may also be effective. However, none of these drugs are intended to improve cognitive function, and there is a need for the development of medicines that can improve cognitive function.

### Citation List

### Non Patent Literature

[Non-Patent Literature 1] Wada-Isoe K., et al., Epidemiological Survey of Frontotemporal Lobar Degeneration in Tottori Prefecture, Japan., Dement. Geriatr. Cogn. Dis. Extra. 2 (1), 381-386, 2012
[Non-Patent Literature 2] Imaizumi K., et al., Rostrocaudal Areal Patterning of Human PSC-Derived Cortical Neurons by FGF8 Signaling., eNeuro, 5 (2), 2018

### Summary of Invention

### Technical Problem

The objective of the present invention is to provide a therapeutic agent or a therapeutic composition for frontotemporal lobar degeneration (FTLD), a disease for which no therapeutic drug has been developed to date, and a method for treating FTLD.

### Solution to Problem

The inventors of the present invention induced the differentiation of frontal cortical neurons from iPS cells derived from FTLD patients that reflect the pathology of FTLD, and then administered some therapeutic agents for FTLD described below to these frontal cortical neurons, and found that the pathology of FTLD in frontal cortical neurons was improved.

Based on this finding, the present invention demonstrates that the frontotemporal lobar degeneration (FTLD) can be treated with a therapeutic agent for FTLD, comprising a compound represented by the following formula (1-1), a compound represented by the formula (2-1), or a compound represented by the formula (3-1), a pharmaceutically acceptable salt thereof, or a solvate thereof.

More specifically, to solve the above-mentioned problems, the present application provides the following embodiments:
[1]: A therapeutic agent for frontotemporal lobar degeneration (FTLD), comprising a compound represented by the following formula (1-1): 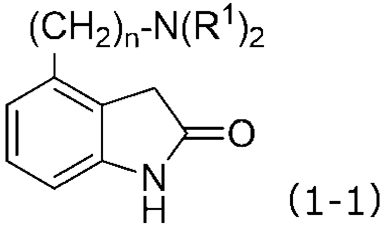
   [in the formula (1-1), R¹ each independently represents an alkyl group having 1 to 6 carbon atoms or a 4-hydroxyphenethyl group, and n represents an integer from 1 to 3], a compound represented by the following formula (2-1): 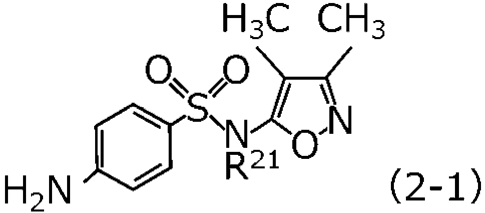
   [in the formula (2-1), R²¹ is selected from the group consisting of hydrogen, fatty acid acyl groups having 2 to 18 carbon atoms, and aromatic carboxylic acid acyl groups having 7 to 9 carbon atoms],
   or a compound represented by the following formula (3-1): 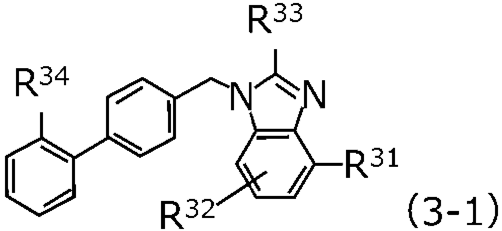
   [in the formula (3-1), R³¹ is selected from the group consisting of hydrogen, halogen, alkyl groups having 1 to 6 carbon atoms, and CF₃;
   R³² is selected from the group consisting of alkoxy groups having 1 to 6 carbon atoms substituted with an imidazolyl group, and optionally substituted nitrogen-containing aromatic heterocycles;
   R³³ is selected from the group consisting of hydrogen, alkyl groups having 1 to 6 carbon atoms, and cycloalkyl groups having 3 to 6 carbon atoms;
   R³⁴ is selected from the group consisting of carboxy groups, cyano groups, and 1H-tetrazolyl groups],
   a pharmaceutically acceptable salt thereof, or a solvate thereof.
[2]: The therapeutic agent for FTLD according to [1], wherein the FTLD is one or more symptoms selected from behavioral variant frontotemporal dementia (bvFTD), semantic dementia (SD), and progressive non-fluent aphasia (PNFA).
[3]: The therapeutic agent for FTLD according to [1] or [2], wherein, in formula (1-1), n is 2.
[4]: The therapeutic agent for FTLD according to [1] or [2], wherein, in the formula (1-1), R¹ is an n-propyl group.
[5]: The therapeutic agent for FTLD according to [3], wherein, in the formula (1-1), R¹ is an n-propyl group.
[6]: The therapeutic agent for FTLD according to [1] or [2], wherein the compound represented by the formula (1-1) is a compound represented by the following formula (1-2): 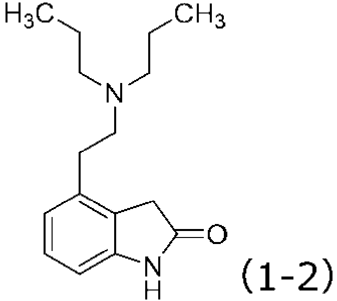 (4-[2-(dipropylamino)ethyl]-1,3-dihydro-2H-indol-2-one).
[7]: The therapeutic agent for FTLD according to [1] or [2], wherein the pharmaceutically acceptable salt of the compound represented by the formula (1-1) is the hydrochloride salt of the compound represented by the following formula (1-2): 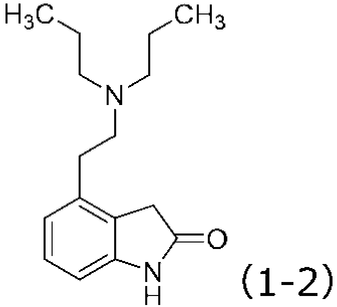 (4-[2-(dipropylamino)ethyl]-1,3-dihydro-2H-indol-2-one hydrochloride).
[8]: The therapeutic agent for FTLD according to [1] or [2], wherein the compound represented by the formula (2-1) is a compound represented by the following formula (2-2): 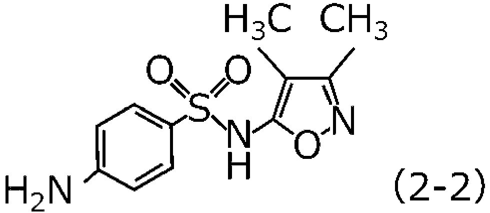 (4-amino-N-(3,4-dimethyl-5-isoxazoyl)benzenesulfonamide).
[9]: The therapeutic agent for FTLD according to [1] or [2], wherein the compound represented by the formula (3-1) is a compound represented by the following formula (3-2): 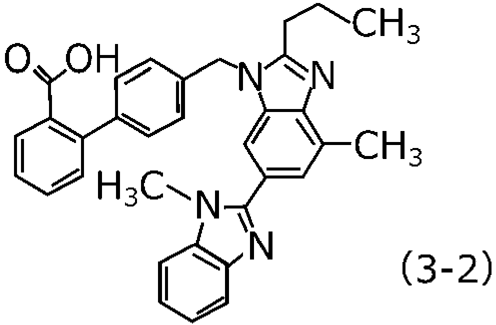 (4'-[[4-methyl-6-(1-methyl-1H-benzimidazol-2-yl)-2-propyl 1H-benzimidazol-1-yl]methyl]biphenyl-2-carboxylic acid).
[10]: A pharmaceutical composition for treating frontotemporal lobar degeneration (FTLD), comprising, as an active ingredient, a compound represented by the following formula (1-1): 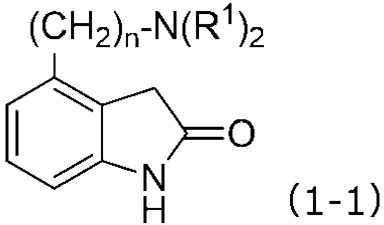
   [in the formula (1-1), R¹ each independently represents an alkyl group having 1 to 6 carbon atoms or a 4-hydroxyphenethyl group, and n represents an integer from 1 to 3], a compound represented by the following formula (2-1): 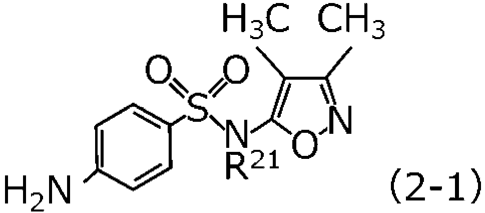
   [in the formula (2-1), R²¹ is selected from the group consisting of hydrogen, fatty acid acyl groups having 2 to 18 carbon atoms, and aromatic carboxylic acid acyl groups having 7 to 9 carbon atoms],
   or a compound represented by the following formula (3-1): 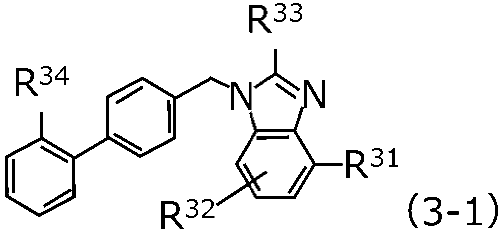
   [in the formula (3-1), R³¹ is selected from the group consisting of hydrogen, halogen, alkyl groups having 1 to 6 carbon atoms, and CF₃;
   R³² is selected from the group consisting of alkoxy groups having 1 to 6 carbon atoms substituted with an imidazolyl group, and optionally substituted nitrogen-containing aromatic heterocycles;
   R³³ selected from the group consisting of hydrogen, alkyl groups having 1 to 6 carbon atoms, and cycloalkyl groups having 3 to 6 carbon atoms;
   R³⁴ selected from the group consisting of carboxy groups, cyano groups, and 1H-tetrazolyl groups],
   a pharmaceutically acceptable salt thereof, or a solvate thereof.

### Advantageous Effects of Invention

The therapeutic agent for FTLD of the present invention comprises a compound obtained by screening compounds using phenotypes characteristic of human pathology as an evaluation parameter based on analysis using frontal cortical neurons differentiated from iPS cells derived from FTLD patients as a pathology model. Therefore, the present invention can provide a therapeutic agent with high therapeutic efficacy against FTLD. Furthermore, the present invention can provide a therapeutic agent for FTLD that is effective against all three subtypes of FTLD pathology.

### Brief Description of Drawings

Figure 1 shows the procedure for inducing and differentiating frontal cortical neurons that can be used to screen compounds for the treatment of FTLD, using disease-specific iPS cells derived from FTLD patient cells.
Figure 2 shows that the frontal cortical neurons that degenerate in the pathology of FTLD were successfully generated using disease-specific iPS cells derived from FTLD patient cells.
Figure 3 shows the results of drug discovery screening using disease-specific iPS cells derived from FTLD patient cells.
Figure 4 shows the procedure for a functional analysis assay of ropinirole (ROPI) using frontal cortical neurons generated using disease-specific iPS cells derived from FTLD patient cells.
Figure 5 shows the results of measuring LDH leakage rate as a parameter of the neuroprotective effect following ropinirole administration, and shows the neuronal cell death phenotype (Lactate Dehydrogenase Assay, LDH assay) (Figure 5 left) and the neuroprotective effect following ropinirole (ROPI) administration (Figure 5 right).
Figure 6 shows the neuronal cell death phenotype (TUJ1 immunostaining) (Figure 6(a)) and the numerical value of the number of surviving neurons (Figure 6(b)) of frontal cortical neurons differentiated from iPS cells derived from a healthy subject and from iPS cells derived from an FTLD patient.
Figure 7 shows the effect of various concentrations of ropinirole (ROPI) on the phenotype of neuronal death (TUJ1 immunostaining).
Figure 8 shows the effect of various concentrations of ropinirole (ROPI) on the phenotype of neuronal death (the number of TUJ1 immunostaining-positive cells) (Figure 8(a)) and the phenotype of neuronal functional recovery (neurite length) (Figure 8(b)).
Figure 9 shows the effect of various concentrations of ropinirole (ROPI) on lysosome enlargement.
Figure 10 shows the results of measuring the improvement rate (%) of LDH leakage when various concentrations of ropinirole were added to frontal cortical neurons differentiated from FTLD patient-derived iPS cells.
Figure 11 shows the results of measuring the fluorescence intensity of SiR-lysosome when various concentrations of ropinirole were added to the culture medium of frontal cortical neurons differentiated from FTLD patient-derived iPS cells.
Figure 12 shows the results of measuring the LDH leakage rate when various concentrations of various D2 receptor agonists were added to the culture medium of frontal cortical neurons differentiated from FTLD patient-derived iPS cells.
Figure 13 shows the results of measuring the improvement rate (%) of LDH leakage when various concentrations of Torin1 were added to the culture medium of frontal cortical neurons differentiated from FTLD patient-derived iPS cells.

### Description of Embodiments

### [Screening of Therapeutic Agent for FTLD and Pharmaceutical Compositions for Treating FTLD]

In the present invention, iPS cells are generated from cells collected from FTLD patients, and frontal cortical neurons are induced to be differentiated from these iPS cells. Frontal cortical neurons that reflect the pathology of FTLD are then used to screen compounds included in a compound library for their ability to improve lysosome function and exhibit neuroprotective effects (i.e., improved neuronal survival). This screening allows for the identification of compounds that ameliorate the pathology of FTLD in frontal cortical neurons and have therapeutic effects against FTLD.

For inducing differentiation into frontal cortical neurons, a method in which differentiation of iPS cells is induced into the cerebral nervous system using Dual Smad inhibition (e.g., LDN-193189 and SB431542) and Wnt inhibition (e.g., XAX939) followed by the addition of Fgf8b (Non-Patent Literature 2) can be used. The inventors' studies demonstrated that treatment of Dual Smad inhibition alone was not enough to induce differentiation of iPS cells into the cerebral nervous system, and that treatment with a Wnt antagonist in addition to Dual Smad inhibition was also ineffective to induce differentiation of iPS cells into the cerebral nervous system, demonstrating that treatments of Dual Smad inhibition and Wnt inhibition are essential for inducing differentiation into the cerebral nervous system.

The compound library used for screening can be of any type. For example, a library of compounds that have been clinically tested for various diseases and whose safety has been confirmed can be used.

In the present invention, as a result of screening a compound library using this method, ropinirole, telmisartan, and sulfisoxazole were selected as compounds that improve lysosome function and exhibit neuroprotective effects (improving neuronal survival rate). Based on this finding, the inventors of the present invention have completed the present invention.

### [Therapeutic Agent for FTLD and Pharmaceutical Compositions for Treating FTLD]

The present invention provides a therapeutic agent for FTLD or a pharmaceutical composition for treating FTLD comprising a compound represented by the following formula (1-1): 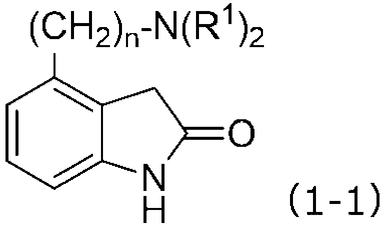
[in the formula (1-1), R¹ each independently represents an alkyl group having 1 to 6 carbon atoms or a 4-hydroxyphenethyl group, and n represents an integer from 1 to 3], a compound represented by the following formula (2-1): 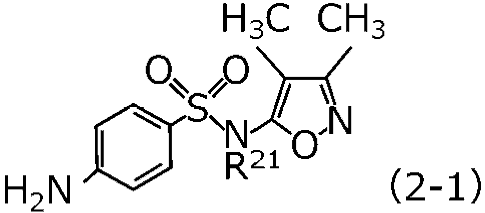
[in the formula (2-1), R²¹ is selected from the group consisting of hydrogen, fatty acid acyl groups having 2 to 18 carbon atoms, and aromatic carboxylic acid acyl groups having 7 to 9 carbon atoms],
or a compound represented by the following formula (3-1): 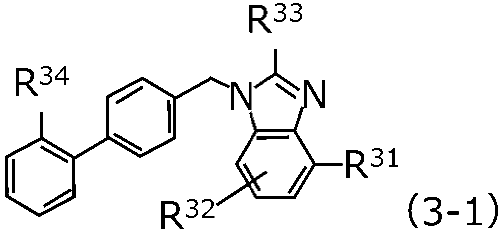
[in the formula (3-1), R³¹ is selected from the group consisting of hydrogen, halogen, alkyl groups having 1 to 6 carbon atoms, and CF₃;
R³² is selected from the group consisting of alkoxy groups having 1 to 6 carbon atoms substituted with an imidazolyl group, and optionally substituted nitrogen-containing aromatic heterocycles;
R³³ is selected from the group consisting of hydrogen, alkyl groups having 1 to 6 carbon atoms, and cycloalkyl groups having 3 to 6 carbon atoms;
R³⁴ is selected from the group consisting of carboxy groups, cyano groups, and 1H-tetrazolyl groups],
a pharmaceutically acceptable salt thereof, or a solvate thereof, as a pharmaceutical use of the above compound, a pharmaceutically acceptable salt thereof, or a solvate thereof.

FTLD diseases that can be treated with the therapeutic agent for FTLD or the pharmaceutical composition for treating FTLD of the present invention may include behavioral frontotemporal dementia (bvFTD), semantic dementia (SD), and progressive non-fluent aphasia (PNFA). Furthermore, any of the following FTLD types can be treated: patients of FTLD-tau in which TAU accumulation is observed, patients of FTLD-TDP43 in which TDP-43 accumulation is observed, and patients FTLD-FUS in which FUS accumulation is observed, classified based on their pathological characteristics. Furthermore, any of the following FTLD conditions can be treated: patients with mutations in the *GRN* gene, *MAPT* gene, *C9ORF72* gene, TARDBP gene, etc, based on the genetic characteristics of FTLD patients.

In the compound of the formula (1-1) above, which is the active ingredient of the therapeutic agent for FTLD or the pharmaceutical composition for treating FTLD of the present invention, in the formula (1-1), n can be 1, 2, or 3. When the active ingredient of the therapeutic agent for FTLD or the pharmaceutical composition for treating FTLD of the present invention is ropinirole, as described below, n in the formula (1-1) is 2. Therefore, the active ingredient of the therapeutic agent for FTLD or the pharmaceutical composition for treating FTLD of the present invention may be a compound in which n in formula (1-1) above is 2.

Furthermore, in the compound of formula (1-1) above, which is the active ingredient of the therapeutic agent for FTLD or the pharmaceutical composition for treating FTLD of the present invention, R¹ in the formula (1-1) above may be a linear, branched, or cyclic alkyl group having 1 to 6 carbon atoms, and more specifically, may be a methyl group, an ethyl group, an n-propyl group, an isopropyl group, an n-butyl group, an isobutyl group, a sec-butyl group, a tert-butyl group, a cyclobutyl group, an n-pentyl group, a cyclopentyl group, an n-hexyl group, and a cyclohexyl group. When the active ingredient of the therapeutic agent for FTLD or the pharmaceutical composition for treating FTLD of the present invention is ropinirole, as described below, R¹ in the formula (1-1) is an n-propyl group. Therefore, the active ingredient of the therapeutic agent for FTLD or the pharmaceutical composition for treating FTLD of the present invention may be a compound in which R¹ in the formula (1-1) above is an n-propyl group.

The compound represented by the formula (1-1) above, which is the active ingredient of the therapeutic agent for FTLD or the pharmaceutical composition for treating FTLD of the present invention, may be 4-[2-(dipropylamino)ethyl]-1,3-dihydro-2H-indol-2-one. That is, the compound represented by the formula (1-1) above may be ropinirole. The chemical formula of ropinirole is shown in the formula (1-2) below.

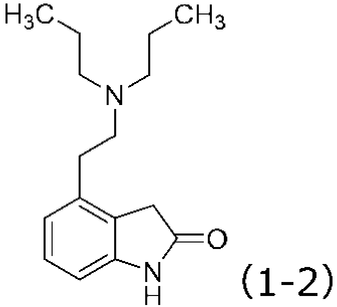

Ropinirole was originally developed as a therapeutic agent for Parkinson's disease due to its dopamine D2 receptor agonist activity in dopaminergic neurons. In the present invention, compared to the mechanism of action of ropinirole against Parkinson's disease, it is currently unclear whether ropinirole acts on FTLD based on the same intracellular mechanism of action or a different intracellular mechanism. However, clinical trials regarding ropinirole have already been completed as a pharmaceutical composition, and its safety when administered to living organisms has been fully confirmed. As such, because ropinirole is an existing drug, it is possible to rapidly develop a therapeutic agent for FTLD or a pharmaceutical composition for treating FTLD.

The compound represented by the formula (2-1) above, which is the active ingredient of the therapeutic agent for FTLD or the pharmaceutical composition for treating FTLD of the present invention, may be 4-amino-N-(3,4-dimethyl-5-isoxazoyl)benzenesulfonamide. That is, the compound represented by the formula (2-1) above may be sulfisoxazole. The chemical formula of sulfisoxazole is shown below in the formula (2-2) below.

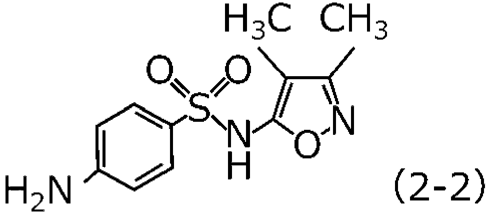

Sulfisoxazole was originally developed as a sulfonamide antibiotics, but in recent years, its therapeutic application for diseases such as Alzheimer's disease and Parkinson's disease, based on its ability to modify cellular stress responses, has been investigated. Compared to the mechanism of action of sulfisoxazole against Alzheimer's disease or Parkinson's disease, it is currently unclear whether sulfisoxazole acts on FTLD based on the same intracellular mechanism of action or a different intracellular mechanism. However, clinical trials regarding sulfisoxazole have already been completed as a pharmaceutical composition, and its safety when administered to living organisms has been fully confirmed. As such, because sulfisoxazole is an existing drug, it is possible to rapidly develop a therapeutic agent for FTLD or a pharmaceutical composition for treating FTLD.

The compound represented by the formula (3-1) above, which is the active ingredient of the therapeutic agent for FTLD or the pharmaceutical composition for treating FTLD of the present invention,

The compound represented by the formula (3-1) above, which is the active ingredient of the therapeutic agent for FTLD or the pharmaceutical composition for treating FTLD of the present invention, may be 4'-[[4-methyl-6-(1-methyl-1H-benzimidazol-2-yl)-2-propyl 1H-benzimidazol-1-yl]methyl]biphenyl-2-carboxylic acid. That is, the compound represented by the formula (3-1) above may be telmisartan. The chemical formula of telmisartan is shown below in the formula (3-2).

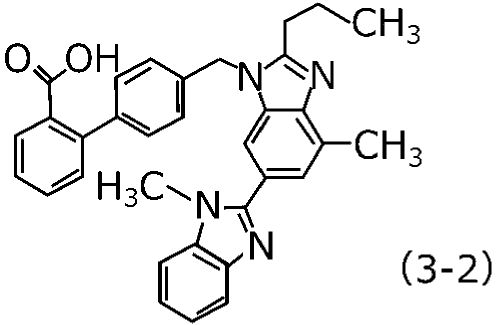

Telmisartan was originally developed as a therapeutic agent for hypertension due to its biliary-excreted, sustained AT1 receptor blocker activity. In the present invention, compared to the mechanism of action of telmisartan against hypertension, it is currently unclear whether telmisartan acts on FTLD based on the same intracellular mechanism of action or a different intracellular mechanism. However, clinical trials regarding telmisartan have already been completed as a pharmaceutical composition, and its safety when administered to the living organisms has been fully confirmed. As such, because telmisartan is an existing drug, it is possible to rapidly develop a therapeutic agent for FTLD or a pharmaceutical composition for treating FTLD.

The active ingredient of the therapeutic agent for FTLD or the pharmaceutical composition for treating FTLD of the present invention may be a salt of any of the compounds represented by the above formula (1-1), (2-1), or (3-1); a solvate of any of the compounds represented by the above formula (1-1), (2-1), or (3-1); or a solvate of a salt of any of the compounds represented by the above formula (1-1), (2-1), or (3-1).

When a salt of any of the compounds represented by the above formula (1-1), (2-1), or (3-1) is used as the active ingredient of the therapeutic agent for FTLD or the pharmaceutical composition for treating FTLD of the present invention, the salt is not particularly limited as long as it is a pharmaceutically acceptable salt, and examples include inorganic acid salts such as hydrochloride, sulfate, hydrobromide, nitrate, and phosphate; organic acid salts such as acetate, mesylate, succinate, maleate, fumarate, citrate, and tartrate; alkali metal salts such as sodium salt and potassium salt; alkaline earth metal salts such as magnesium salt and calcium salt; metal salts such as aluminum salt and zinc salt; ammonium salts such as ammonium salt and tetramethylammonium salt; organic amine addition salts such as morpholine and piperidine; and amino acid addition salts such as glycine, phenylalanine, lysine, aspartic acid, and glutamic acid.

Furthermore, when a solvate of any of the compounds represented by the above formula (1-1), (2-1), or (3-1) above or a salt thereof is used in the therapeutic agent for FTLD or the pharmaceutical composition for treating FTLD of the present invention, the solvate is not particularly limited as long as it is a pharmaceutically acceptable solvate, and examples include hydrates, organic solvates, etc.

The active ingredient of the therapeutic agent for FTLD or the pharmaceutical composition for treating FTLD of the present invention may be 4-[2-(dipropylamino)ethyl]-1,3-dihydro-2H-indol-2-one hydrochloride, i.e., ropinirole hydrochloride.

The active ingredient of the therapeutic agent for FTLD or the pharmaceutical composition for treating FTLD of the present invention may be 4-amino-N-(3,4-dimethyl-5-isoxazoyl)benzenesulfonamide, i.e., sulfisoxazole.

The active ingredient of the therapeutic agent for FTLD or the pharmaceutical composition for treating FTLD of the present invention may be 4'-[[4-methyl-6-(1-methyl-1H-benzimidazol-2-yl)-2-propyl 1H-benzimidazol-1-yl]methyl]biphenyl-2-carboxylic acid, i.e., telmisartan.

The pharmaceutical composition for treating FTLD of the present invention may be formulated as a pharmaceutical composition and can be administered orally in the form of, for example, tablets, capsules, elixirs, microcapsules, etc., or parenterally in the form of injections, suppositories, topical skin preparations, etc. More specifically, topical skin preparations include dosage forms such as ointments and patches.

In the pharmaceutical composition for treating FTLD of the present invention, any pharmaceutically acceptable carriers typically used in the formulation of pharmaceutical compositions can be used, without particular limitation. More specifically, examples of carriers may include binders such as hypromellose, dextrin, macrogol 400, gelatin, corn starch, tragacanth gum, and gum arabic; excipients such as lactose hydrate, D-mannitol, starch, crystalline cellulose, and alginic acid; solvents for injections such as water, ethanol, and glycerin; and adhesives such as rubber-based adhesives and silicone-based adhesives.

The pharmaceutical composition for treating FTLD of the present invention may contain additives. Examples of additives may include lubricants such as calcium stearate and magnesium stearate; sweeteners such as sucrose, lactose, saccharin, and maltitol; flavoring agents such as peppermint and akamono oil; stabilizers such as carmellose sodium, hydrogenated oil, light anhydrous silicic acid, povidone, glycerin fatty acid ester, benzyl alcohol, and phenol; buffering agents such as phosphate and sodium acetate; solubilizers such as benzyl benzoate and benzyl alcohol; and coloring agents such as yellow ferric oxide, red ferric oxide, black ferric oxide, and titanium oxide.

The pharmaceutical composition for treating FTLD of the present invention can be formulated by appropriately combining the above-mentioned active ingredients with the above-mentioned pharmaceutically acceptable carriers and additives, and mixing them in a unit dosage form required for generally accepted pharmaceutical practice. The pharmaceutical composition for treating FTLD of the present invention may comprise one active ingredient, or two or more active ingredients in combination.

In general, an appropriate daily dose of a pharmaceutical composition for treating FTLD of the present invention is an amount containing the effective minimum dose of the active ingredient to exhibit a therapeutic effect. This effective minimum dose is determined based on various factors, including the activity of the active ingredient contained in the pharmaceutical composition for treating FTLD, functional group modification(s) that determine lipid solubility or water solubility, the route of administration, the duration of administration, the excretion rate of the specific active ingredient used, the duration of treatment, other drugs, compounds, and/or substances used in combination therewith, age, sex, body weight, disease, medical condition, and medical history of the patient, and other factors well known in the medical arts. Typically, the dose of the pharmaceutical composition for treating FTLD of the present invention administered to a patient is an amount including the effective ingredient at about 0.0001 to about 100 mg/kg of body weight per day. The pharmaceutical composition for treating FTLD of the present invention may be administered once a day or in divided doses about 2 to 4 times a day.

In particular, in a case where the active ingredient is a compound represented by the formula (1-2), the dosage of the pharmaceutical composition for treating FTLD of the present invention is considered to be orally administered at a dose of 2 mg of the active ingredient once day, for example, as a sustained-release ropinirole formulation, and is administered orally, with the dose increased weekly to no more than 16-24 mg of active ingredient per day.

In particular, in a case where the active ingredient is a compound represented by the formula (2-2), the dosage of the pharmaceutical composition for treating FTLD of the present invention is considered to be orally administered at a dose of 4,000 mg of, for example, sulfisoxazole as the active ingredient in 4 to 6 divided doses per day, and is gradually increasing the dose so as not to exceed a daily dose of 8,000 mg of the active ingredient.

In particular, in a case where the active ingredient is a compound represented by the formula (3-2), the dosage of the pharmaceutical composition for treating FTLD of the present invention is considered to be orally administered at a dose of 20 mg once a day, for example, as a sustained-release telmisartan formulation, and is gradually increasing the dose so as not to exceed a daily dose of 80 mg of the active ingredient.

### [Other Embodiments]

In another embodiment, the present invention provides a method for treating FTLD, comprising administering to a patient in need of such treatment any one of the compounds represented by the formula (1-1), (2-1), or (3-1), or a pharmaceutically acceptable salt thereof, or a solvate thereof. In this embodiment of the present invention, the active ingredient may be any one of the compounds represented by the formula (1-1), (2-1), or (3-1), or a pharmaceutically acceptable salt thereof, or a solvate thereof, as described above. Furthermore, in this embodiment of the present invention, the dosage of the active ingredient can be determined by considering the appropriate dosage for each compound. For example, the dose of ropinirole used as the active ingredient is as described above.

The present invention provides a compound represented by the above formula (1-1), a compound represented by the above formula (2-1), or a compound represented by the above formula (3-1), or a pharmaceutically acceptable salt thereof, or a solvate thereof, for treating FTLD. In this embodiment of the present invention, the compound represented by the above formula (1-1), a compound represented by the above formula (2-1), or a compound represented by the above formula (3-1), or a pharmaceutically acceptable salt thereof, or a solvate thereof, can be the same as those described above.

The following examples illustrate the present invention in more detail. The following examples are not intended to limit the present invention in any way.

### Examples

### Example 1: Screening for Compounds for Treating FTLD

In this example, disease-specific iPS cells derived from FTLD patient cells were used to induce differentiation of frontal cortical neurons that can be used to screen for compounds for treating FTLD and compounds that can ameliorate the pathology of FTLD in these frontal cortical neurons were then screened.

### (1-1) Induction of Differentiation of Frontal Cortical Neurons

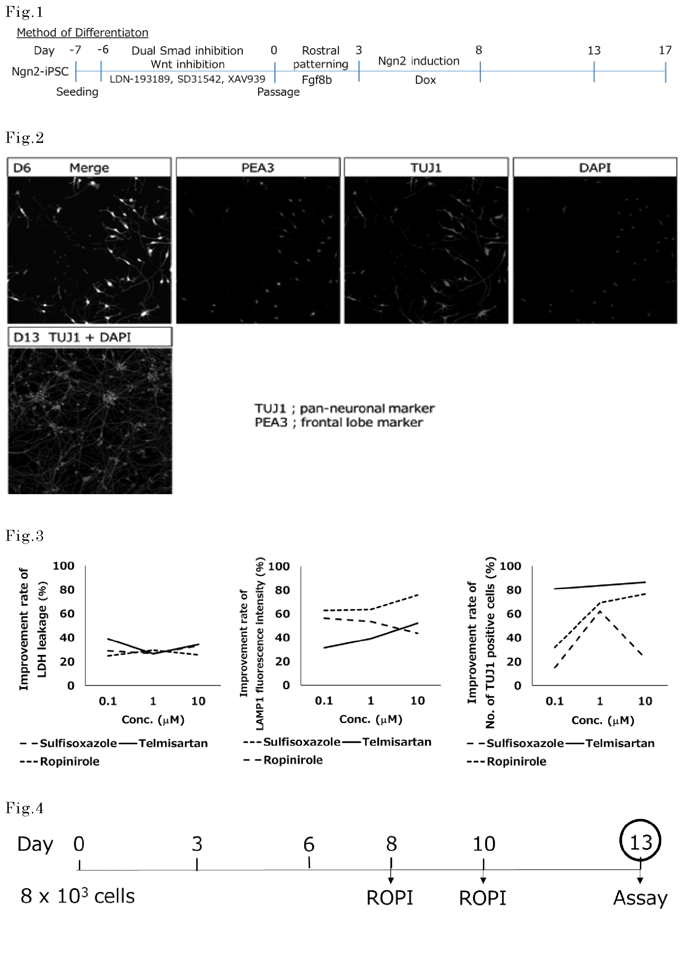

It is known that familial FTLD includes pathological condition caused by mutations in the *GRN* gene, pathological condition caused by mutations in the *MAPT* gene, or pathological condition caused by mutations in the *C9ORF72* gene. Therefore, iPS cells derived from a healthy individual or iPS cells derived from an FTLD patient with the *GRN* gene mutation (a bvFTD patient with the GRN^{S116X} mutation), as a representative familial FTLD, were differentiated into frontal cortical neurons using the culture method shown in Figure 1. The *GRN* gene mutation was S116X.

Specifically, each cell line (Ngn2-iPSC in Figure 1) which was transformed with a plasmid carrying the *Ngn2* gene linked to a promoter whose expression is induced in the presence of DOX using the PiggyBac method, was cultured for six days in a culture medium containing a final concentration of 50 nM LDN-193189 (CAS No.: 1062368-24-4), a final concentration of 2 microM SB431542 (CAS No.: 301836-41-9), and a final concentration of 1 microM XAV939 (CAS No.: 284028-89-3) to induce cortical neural stem cells. The medium was changed every two to three days.

The resulting cortical neural stem cells were then dissociated into individual cells, which are cultured for three days in a culture medium containing Fgf8b.

Furthermore, the cells were induced with a final concentration of 0.25 ng/mL doxycycline (CAS No.: 24390-14-5) to express NGN2 protein, a transcription factor expressed in the early stages of neuronal differentiation and, at the same time, were cultured for five days in a culture medium containing a final concentration of 3 microM DAPT (CAS No.: 208255-80-5) and a final concentration of 2 microM palbociclib (CAS No.: 571190-30-2). The medium was changed every two to three days.

The differentiated frontal cortical neurons were immunostained using anti-beta III tubulin antibody (Chemicon) and anti-Pea3 antibody (Abcam) to examine the expression of beta III-tubulin and polyomavirus enhancer activator 3 (Pea3). The results are shown in Figure 2. The resulting neurons were confirmed the frontal lobe marker PEA3 expression using anti-Pea3 (Abcam) antibody on D6 (based on the days of culture depicted in Figure 1), and the pan-neuronal marker TUJ1 expression using anti-beta III tubulin antibody (Chemicon) on D13 (based on the days of culture depicted in Figure 1), confirming that the resulting cells were frontal cortical neurons.

### (1-2) Results of Drug Discovery Screening Using Frontal Cortical Neurons

Using frontal cortical neurons derived from FTLD disease-specific iPS cells derived from an FTLD disease line (a bvFTD patient with the GRN^{S116X} mutation) differentiated as described in Example 1 (1-1), we screened existing drug libraries for drugs that recover the FTLD phenotype (closer to the wild-type phenotype), by lactate dehydrogenase (LDH) leakage and the number of TUJ1-positive cells, a marker of neuronal death, as well as by the fluorescence intensity of LAMP1 immunostaining, a marker of lysosome abnormalities, as indicators. LDH leakage was measured by a commercially available kit (model number "G7891," Promega). The number of TUJ1-positive cells was determined by immunostaining with an anti-Beta III tubulin antibody (Chemicon), and LAMP1 immunostaining, a marker of lysosome abnormalities, was performed using an anti-LAMP1 antibody [H4A3] (Abcam).

Compounds included in the existing drug library were added to the culture medium from day 13 to day 17 after the start of differentiation induction, and screening was performed. As a result, three drugs, ropinirole, telmisartan, and sulfisoxazole, were identified as promising FTLD treatments. Figure 3 shows the improvement rate (%) of the FTLD phenotype when these drugs were added to the culture medium.

The improvement rate (%) of the FTLD phenotype was calculated using the following formula (1) for each parameter:$Improvement rate \left(%\right) = \left(A⋅B\right) / \left(A⋅C\right) \times 100$
[wherein, in the formula (1),
"A" represents the measured value of frontal cortical neurons induced to differentiate from iPS cells derived from an FTLD patient in the absence of drug,
"B" represents the measured value of frontal cortical neurons induced to differentiate from iPS cells derived from an FTLD patient in the presence of drug,
"C" represents the measured value of frontal cortical neurons induced to differentiate from iPS cells derived from a healthy individual in the absence of drug.]

Figure 3 shows the results of adding each three drugs obtained above (ropinirole, telmisartan, or sulfisoxazole) at a final concentration of 100 nM, 1 microM, or 10 microM to the culture medium of frontal cortical neurons differentiated from iPS cells derived from FTLD patients. The results demonstrated that all three drugs exerted neuroprotective effect and lysosomal function-improving effect at concentrations ranging from 100 nM to 10 microM. Of these three drugs, ropinirole was further investigated in the following example.

### Example 2: Effect of Ropinirole

In this example, a functional analysis assay of ropinirole obtained in Example (1-2) was performed using frontal cortical neurons generated using disease-specific iPS cells derived from FTLD patient cells.

### (2-1) Cell Treatment Procedure with Ropinirole

The efficacy of ropinirole was evaluated in the same manner as in Example 1-2, except that the timing of ropinirole addition to the culture was changed to period from day 8 to day 13 after the start of differentiation induction, as shown in Figure 4.

Specifically, ropinirole was added to the culture medium of frontal cortical neurons differentiated from iPS cells derived from an FTLD patient with a mutation in the *GRN* gene (GRN-FTLD) on days 8 and 10, and assays were performed on day 13. Ropinirole was added to the culture medium at final concentrations of 10 nM, 30 nM, 100 nM, or 300 nM.

### (2-2) Neuroprotective Effect of Ropinirole

As a parameter of neuroprotective effect, the rate of LDH leakage following ropinirole administration was measured. Measurements of the LDH leakage rate were performed using a commercially available kit (model number "G7891", Promega) as in Example 1 (1-2). The results are shown in Figure 5.

The left graph of Figure 5 is a graph showing the results of measuring the LDH leakage rate in frontal cortical neurons differentiated from iPS cells derived from healthy individuals and iPS cells derived from a GRN^{S116X} FTLD patient. In the left graph of Figure 5, the vertical axis shows the LDH leakage rate (relative value) of frontal cortical neurons differentiated from iPS cells derived from FTLD patients, calculated based on the average LDH leakage rate in frontal cortical neurons differentiated from iPS cells derived from healthy individuals. "**," "##," and "&&" indicate significant differences at a risk level of less than 1% between GRN^{S116X} FTLD patient-derived cortical neurons and healthy individual-derived cortical neurons (RC802, ND025, ND554).

In contrast, the right graph of Figure 5 shows the results of measuring the LDH leakage rate when various concentrations of ropinirole (30 nM, 100 nM, 300 nM, or 1 microM) was added to the culture medium of frontal cortical neurons differentiated from iPS cells derived from a GRN^{S116X} FTLD patient. In the right graph of Figure 5, the vertical axis shows the LDH leakage rate (relative value) calculated based on the LDH leakage rate in the absence of ropinirole (0 nM), and the horizontal axis shows the results when ropinirole was added to the culture medium at 30 nM, 100 nM, 300 nM, or 1 microM. In the right graph of Figure 5, "**" indicates a significant difference at a risk level of less than 1%.

As a result, neuroprotective effect (reduced LDH leakage) was observed with the addition of ropinirole, even day 8 to day 13 after the start of differentiation induction. Furthermore, the EC₅₀ of ropinirole was 38.1 nM (calculated using ImageJ and Excel), indicating that ropinirole can exert neuroprotective effect at a very low dose.

### (2-3) Effect of Ropinirole on Neuronal Cell Death

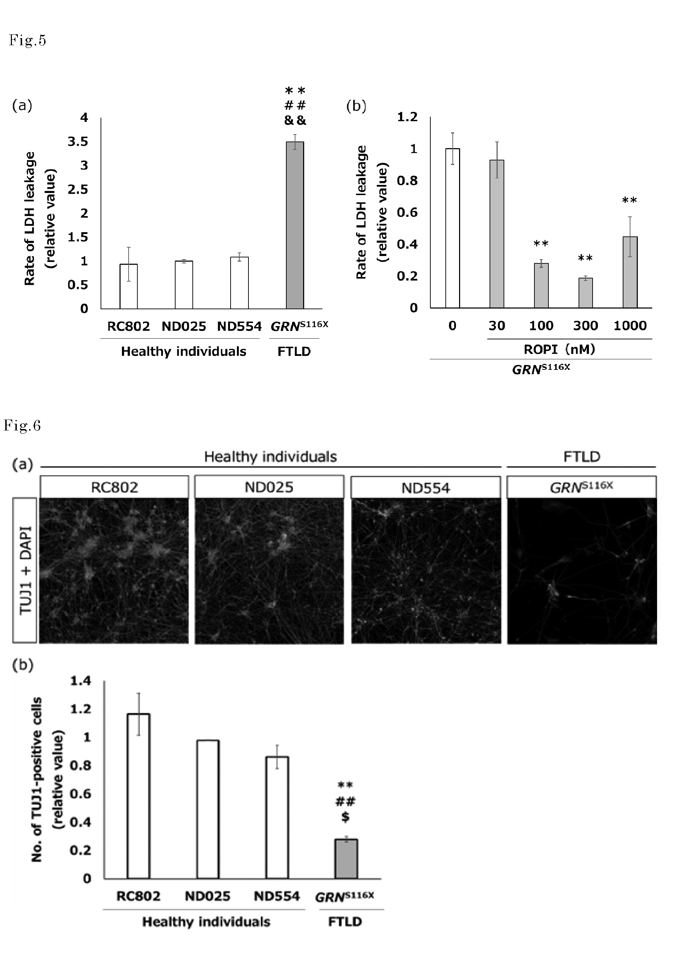

First, we examined phenotype of the neuronal cell death using TUJ1 expression as a parameter in frontal cortical neurons differentiated from iPS cells derived from healthy individuals and frontal cortical neurons differentiated from iPS cells derived from FTLD patients (by TUJ1 immunostaining). TUJ1 immunostaining was performed using Anti-Beta III Tubulin Antibody (Chemicon) as in Example 1 (1-1). The results are shown in Figure 6.

Specifically, Figure 6 shows the results of TUJ1 immunostaining of frontal cortical neurons differentiated from iPS cells derived from healthy individuals and those differentiated from iPS cells derived from FTLD patients (Figure 6(a)), and a graph showing the numerical values of the number of surviving neurons based on the results of Figure 6(a) (Figure 6(b)). In Figure 6(b), the vertical axis shows the number of TUJ1-positive cells (relative value) in frontal cortical neurons differentiated from iPS cells derived from FTLD patients, calculated based on the average number of TUJ1-positive cells in frontal cortical neurons differentiated from iPS cells derived from healthy individuals. The symbols "**," "##," and "&" indicate significant differences between cortical neurons derived from GRN^{S116X} FTLD patient and those derived from healthy individual-derived cortical neurons (RC802, ND025, and ND554) at a risk level of a less than 1%, less than 1%, and less than 5%, respectively.

As a characteristic of frontal cortical neurons differentiated from iPS cells, neuronal death (a decrease in the number of TUJ1-positive cells) was observed in frontal cortical neurons induced to differentiate from iPS cells derived from GRN^{S116X} FTLD patients on days 8 to 13 after the start of differentiation induction.

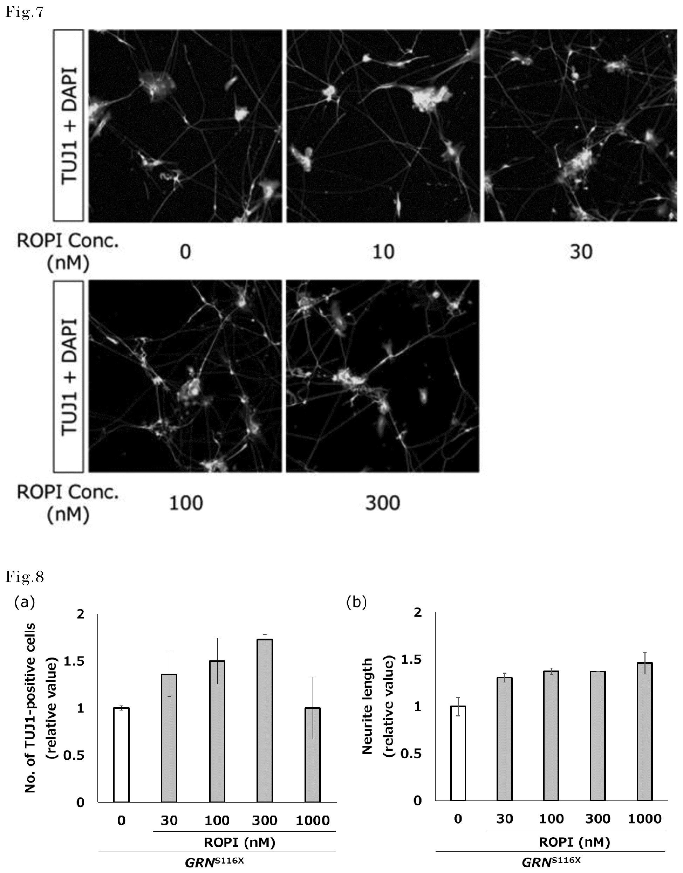

Next, ropinirole was added to the culture medium of frontal cortical neurons differentiated from iPS cells derived from an FTLD patient with a GRN gene mutation (GRN^{S116X} FTLD) on days 8 to 13 after the start of differentiation induction, and the number of TUJ1-positive cells as a parameter of neuronal death, and neurite length as a parameter of neuronal function, were measured. Ropinirole was added to the culture medium at final concentrations of 30 nM, 100 nM, 300 nM, and 1 microM. The results are shown in Figures 7 and 8.

Figure 7 shows the images of TUJ1 immunostaining when ropinirole was added to the culture medium of frontal cortical neurons differentiated from iPS cells derived from a GRN^{S116X} FTLD patient. "ROPI" indicates the result of adding ropinirole to the culture medium at each concentration. These results demonstrate that ropinirole has neuroprotective effects.

Furthermore, when various concentrations of ropinirole were added to the culture medium of frontal cortical neurons differentiated from iPS cells derived from a GRN^{S116X} FTLD patient, the characteristics of the cerebral cortical neurons were examined using the number of TUJ1-positive cells and neurite length as parameters. The results are shown in Figure 8.

Figure 8(a) is a graph showing the results of measuring the number of TUJ1-positive cells when various concentrations of ropinirole were added to the culture medium of frontal cortical neurons differentiated from iPS cells derived from a GRN^{S116X} FTLD patient. In Figure 8(a), the vertical axis shows the number of TUJ1-positive cells (relative value) calculated based on the number of TUJ1-positive cells without ropinirole (0 nM). "ROPI" indicates the results of adding ropinirole to the culture medium at each concentration.

Figure 8(b) is a graph showing the results of measuring the neurite length when various concentrations of ropinirole were added to the culture medium of frontal cortical neurons differentiated from iPS cells derived from a GRN^{S116X} FTLD patient. In Figure 8(b), the vertical axis shows the neurite length (relative value) calculated based on the neurite length without ropinirole (0 nM). "ROPI" indicates the result of adding ropinirole to the medium at each concentration.

These results indicate that the addition of ropinirole between days 8 and 13 after the start of differentiation induction resulted in neuroprotection effect (i.e., an effect of increasing the number of TUJ1-positive cells) and in neuronal function recovery effect (i.e., an effect of increasing neurite length). In other words, the neuroprotective effect of ropinirole was demonstrated by methods other than the LDH assay.

### (2-4) Effect of ROPI on Lysosome enlargement

Lysosome enlargement is known to be a characteristic of neurons in FTLD disease. The inventors investigated whether the addition of ropinirole altered lysosome enlargement. The efficacy of ropinirole was evaluated by immunostaining for LAMP1, a marker of lysosome abnormalities, as described in Example 1 (1-2), except that ropinirole was added between days 8 and 13 after the start of differentiation induction.

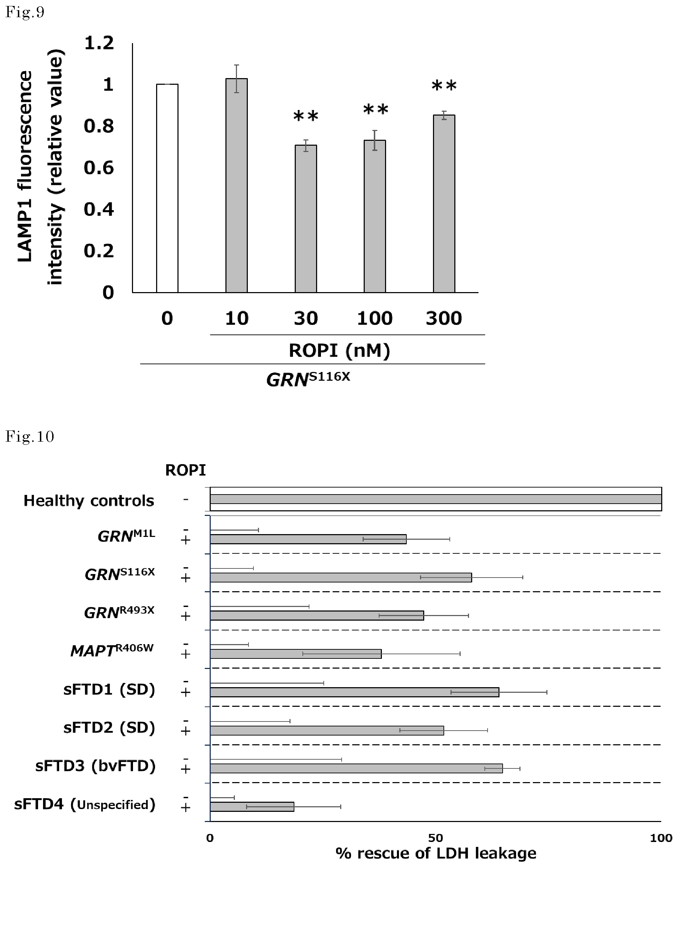

Specifically, various concentrations of ropinirole were added to the culture medium of frontal cortical neurons differentiated from iPS cells derived from an FTLD patient with a mutation (S116X) in the GRN gene (GRN^{S116X} FTLD). The fluorescence intensity of LAMP1 immunostaining was measured as a membrane protein in the lysosome inner membrane. Measurement of the fluorescence intensity of LAMP1 immunostaining was performed using anti-LAMP1 antibody [H4A3] (Abcam) as in Example 1 (1-2). Ropinirole was added to the culture medium at final concentration of 10 nM, 30 nM, 100 nM, or 300 nM. The results are shown in Figure 9.

Figure 9 is a graph showing the results of measuring the fluorescence intensity of LAMP1 immunostaining when various concentrations of ropinirole were added to the culture medium of frontal cortical neurons differentiated from iPS cells derived from an FTLD patient. In Figure 9, the vertical axis shows the LAMP1 fluorescence intensity (relative value) calculated based on the LAMP1 fluorescence intensity without ropinirole (0 nM). The horizontal axis shows the concentration of ropinirole added. "ROPI" indicates the results of adding ropinirole to the medium at each concentration. In Figure 9, the symbol "**" indicates that there is a significant difference at a risk level of less than 1%.

As a result, even on days between 8 to 13 after the start of differentiation induction, the addition of ropinirole improved lysosomal function (i.e., reduced LAMP1 fluorescence intensity), and the EC₅₀ of ropinirole was 16.7 nM.

### Example 3: Studies Using Various FTLD Cell Lines

In this example, the effect of ropinirole on neuronal cell death was investigated using disease-specific iPS cells derived from FTLD patients with various causes.

### (3-1) Neuronal Cell Death Inhibitory Effect of Ropinirole

Using a method similar to Example 1 (1-2), multiple iPS cells derived from any of familial (gene mutation) FTLD patients (GRNM1L FTLD, GRNS116X FTLD, GRNR493X FTLD, MAPT^{R406W} FTLD, one patient for case each) and iPS cells derived from sporadic FTLD patients (two SD cases (sFTD1, sFTD2), one bvFTD case (sFTD3), and one case with unknown clinical symptoms (sFTD4)) were differentiated into frontal cortical neurons. Various concentrations of ropinirole were added to the culture medium of each type of frontal cortical neurons differentiated from FTLD patient-derived iPS cells on days 8-13 after the start of differentiation induction. LDH leakage rate, a parameter of neuronal death, was measured using a commercially available kit (model number "G7891"; Promega). Ropinirole was added to the medium at final concentrations of 10 nM, 30 nM, 100 nM, and 300 nM. Measurements of LDH leakage were performed 3-5 times for each experimental condition. Based on these measurement results, the LDH leakage improvement rate (relative value) was calculated from the LDH leakage value before ropinirole application (0 nM) to the LDH leakage value after ropinirole application (300 nM) on the basis of the LDH leakage value in the healthy control sample as the reference (100%). The improvement rate (%) of LDH leakage was calculated as in Example 1 (1-2). The results are shown in Figure 10.

Figure 10 shows the results of measuring the LDH leakage improvement rate when various concentrations of ropinirole were added to the culture medium of frontal cortical neurons differentiated from FTLD patient-derived iPS cells. In Figure 10, the symbols "*" and "**" indicate significant differences at a risk level of less than 5% or less than 1%, respectively, in one-way ANOVA with post-hoc Tukey's test.

As a result, neuroprotective effects (reduced LDH leakage) were observed in frontal cortical neurons derived from multiple FTLD disease iPS cells.

These results support the idea that ropinirole is effective in treating not only the S116X mutation in the GRN gene, but also other familial (genetic mutation) FTLD and sporadic FTLD.

### (3-2) Effect of ROPI on CatD Activity

Since FTLD neurons generate abnormal lysosome function, the present inventors analyzed the mechanism of action of ropinirole by examining the activity of cathepsin D, one of the major proteolytic enzymes present in lysosomes, upon addition of ropinirole. In this experiment, ropinirole treatment was performed as described in Example 1 (1-2), except that ropinirole was added between days 8 and 13 after the start of differentiation induction.

Specifically, frontal cortical neurons differentiated from iPS cells derived from an FTLD patient with a mutation in the GRN gene (S116X) (GRN^{S116X} FTLD) were cultured with various concentrations of ropinirole and the fluorescence intensity of the SiR-Lysosome kit (Cytoskeleton Inc.), a fluorescent dye that is cleaved by CatD developing fluorescence, was measured. The fluorescence intensity of SiR-lysosome was measured in the same manner as described in Example 1 (1-2). Ropinirole was added to the medium at final concentrations of 10 nM, 30 nM, 100 nM, and 300 nM, and measurements were performed for each concentration (n = 4). The results are shown in Figure 11.

Figure 11 is a graph showing the results of measuring SiR-lysosome fluorescence intensity in frontal cortical neurons differentiated from FTLD patient-derived iPS cells when various concentrations of ropinirole were added to the culture medium. In Figure 11, the vertical axis represents SiR-lysosome fluorescence intensity (relative value) calculated based on the SiR-lysosome fluorescence intensity without ropinirole (0 nM). The horizontal axis represents the concentration of ropinirole added. "ROPI" indicates the results obtained when ropinirole was added to the culture medium. In Figure 11, the symbol "*" indicates a significant difference at a risk level of less than 5% based on one-way ANOVA with post-hoc Tukey's test.

As a result, even on days 8 to 13 after the start of differentiation induction, the addition of ropinirole improved CatD activity (increased SiR-lysosome fluorescence intensity).

### (3-3) Analysis of the Mechanism of Ropinirole's Neuroprotective Effect through DRD2-Dependency/Independency Analysis

Since ropinirole was originally known to have dopamine D2 receptor (D2R) agonist activity in dopamine neurons, the inventors used the D2 receptor agonists bromocriptine, rotigotine, or sumanirole to compare the neuroprotective effects of bromocriptine, rotigotine, or sumanirole with those of ropinirole.

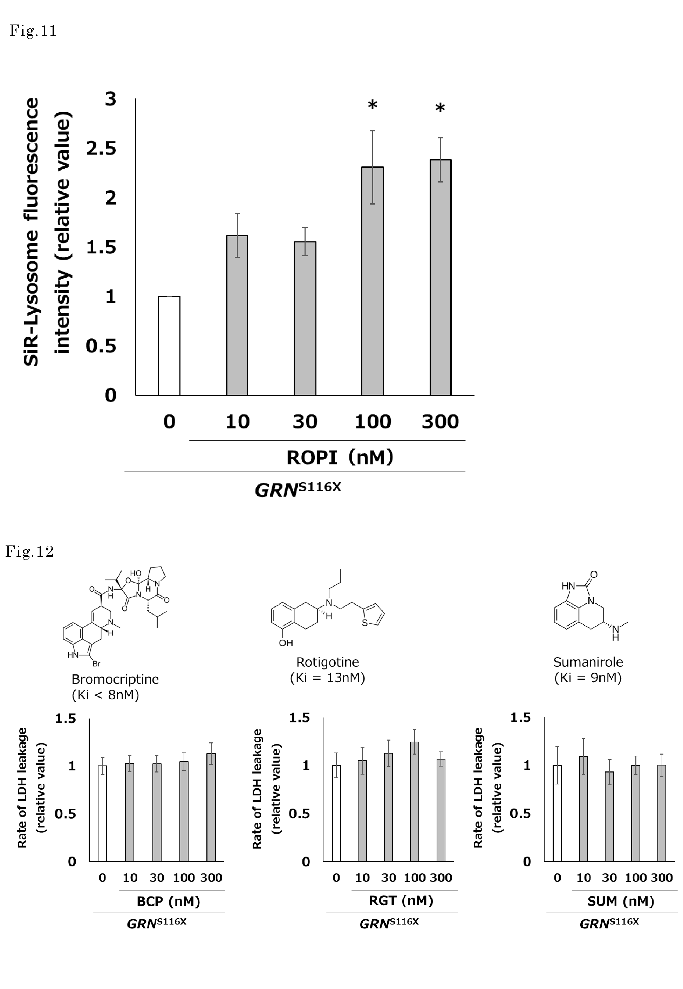

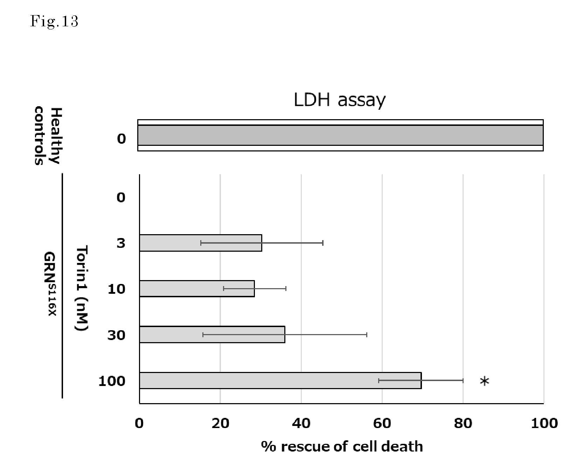

As a parameter of neuroprotection, the LDH leakage rate following administration of ropinirole or various D2 receptor agonists (bromocriptine, rotigotine, or sumanirole) was measured. LDH leakage rate measurements were performed using a commercially available kit (model "G7891," Promega) as described in Example 1 (1-2). The results are shown in Figure 12.

Figure 12 shows the results of measuring LDH leakage rates in frontal cortical neurons differentiated from GRN^{S116X} FTLD patient-derived iPS cells when various concentrations (10 nM, 30 nM, 100 nM, 300 nM) of bromocriptine, rotigotine, or sumanirole were added to the culture medium. In the right center of Figure 5, the vertical axis shows the LDH leakage rate (relative value) calculated based on the LDH leakage rate in the absence of each substance (0 nM), and the horizontal axis shows the results when bromocriptine, rotigotine, or sumanirole was added to the culture medium at concentrations of 10 nM, 30 nM, 100 nM, or 300 nM.

As a result, from day 8 to 13 after the start of differentiation induction, the addition of various D2 receptor agonists did not result in neuroprotective effects (reduced LDH leakage), which was exhibited by the addition of ropinirole.

### (3-4) Analysis of the Mechanism of Ropinirole's Neuroprotective Effect Using an mTOR Inhibitor

The inventors hypothesized that ropinirole exhibits effects such as neuronal cell death inhibition by inhibiting mTOR itself or its signaling pathway. To confirm this effect, it would be desirable to demonstrate that the neuronal cell death inhibition effects of ropinirole are canceled when an mTOR activating agent is added. However, there is a problem in that no suitable mTOR activating agent exists. Therefore, to help demonstrate the mechanism of action of ropinirole, the inventors indirectly inferred that ropinirole inhibits mTOR based on whether an mTOR inhibitor has the same neuronal cell death inhibition effect as ropinirole.

Rapamycin is well known as an mTOR inhibitor in the art, but Rapamycin is known to only partially inhibit mTOR function (i.e., it can completely inhibit S6K, a downstream protein of mTOR, but can not completely inhibit 4EBP, another downstream protein). The effect is thought to be dependent on the mechanism of mTOR inhibition by rapamycin.

On the other hand, second-generation mTOR inhibitors are known to competitively bind to the ATP-binding site required for mTOR kinase activity, thereby almost completely suppressing mTOR kinase activity. Therefore, in this example, the inventors conducted experiments using TORIN1, a second-generation mTOR inhibitor.

As a parameter of neuroprotective effect, the LDH leakage rate due to neuronal cell death was measured after administration of Torin1, which has the following chemical formula:

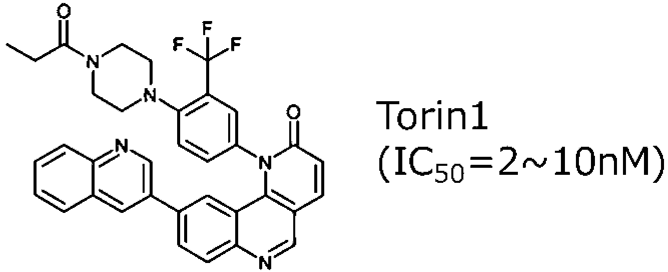

For each experimental condition, n=3 independent experiments were conducted to measure LDH leakage. Based on these measurement results, the LDH leakage improvement rate (relative value) was calculated from the LDH leakage value before Torin1 application (0 nM) to the LDH leakage value after Torin1 application (300 nM), using the LDH leakage value in a healthy control sample as the baseline (100%). The LDH leakage rate was measured using a commercially available kit (model "G7891", Promega) in the same manner as described in Example 1 (1-2). The results are shown in Figure 13.

Figure 13 is a graph showing the results of measuring the LDH leakage improvement rate when various concentrations of Torin1 (Selleck) (3 nM, 10 nM, 30 nM, 100 nM) were added to the culture medium of frontal cortical neurons differentiated from iPS cells derived from a GRN^{S116X} FTLD patient. In Figure 13, the symbol "*" indicates a significant difference at a risk level of less than 5% based on one-way ANOVA with post-hoc Tukey's test.

As a result, the addition of Torin1 from days 8 to 13 after the start of differentiation induction demonstrated a neuroprotective effect (reduced LDH leakage) comparable to that of ropinirole. Therefore, it was suggested that the mechanism of action of ropinirole is mediated by mTOR inhibition.

### Industrial Applicability

The present invention can provide a therapeutic agent for FTLD and a pharmaceutical composition for treating FTLD. The therapeutic agent for FTLD or the pharmaceutical composition for treating FTLD of the present invention can treat not only familial FTLD but also sporadic FTLD. Furthermore, by analyzing the mechanism of drug action of the therapeutic agent for FTLD of the present invention on frontal cortical neurons differentiated from iPS cells derived from FTLD patients, the pathological mechanism of FTLD can be elucidated.

## Claims

1. A therapeutic agent for frontotemporal lobar degeneration (FTLD), comprising a compound represented by the following formula (1-1): 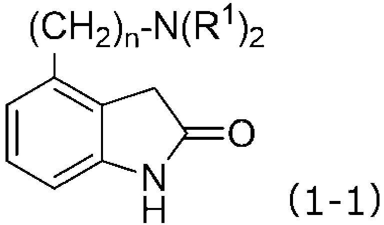
[in the formula (1-1), R¹ each independently represents an alkyl group having 1 to 6 carbon atoms or a 4-hydroxyphenethyl group, and n represents an integer from 1 to 3], a compound represented by the following formula (2-1): 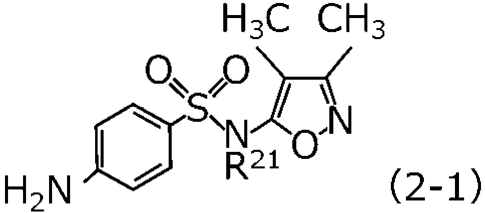
[in the formula (2-1), R²¹ is selected from the group consisting of hydrogen, fatty acid acyl groups having 2 to 18 carbon atoms, and aromatic carboxylic acid acyl groups having 7 to 9 carbon atoms],
or a compound represented by the following formula (3-1): 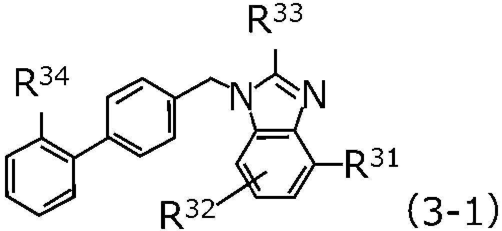
[in the formula (3-1), R³¹ is selected from the group consisting of hydrogen, halogen, alkyl groups having 1 to 6 carbon atoms, and CF₃;
R³² is selected from the group consisting of alkoxy groups having 1 to 6 carbon atoms substituted with an imidazolyl group, and optionally substituted nitrogen-containing aromatic heterocycles;
R³³ is selected from the group consisting of hydrogen, alkyl groups having 1 to 6 carbon atoms, and cycloalkyl groups having 3 to 6 carbon atoms;
R³⁴ is selected from the group consisting of carboxy groups, cyano groups, and 1H-tetrazolyl groups],
a pharmaceutically acceptable salt thereof, or a solvate thereof.

2. The therapeutic agent for FTLD according to claim 1, wherein the FTLD is one or more symptoms selected from behavioral variant frontotemporal dementia (bvFTD), semantic dementia (SD), and progressive non-fluent aphasia (PNFA).

3. The therapeutic agent for FTLD according to claim 1 or 2, wherein, in formula (1-1), n is 2.

4. The therapeutic agent for FTLD according to claim 1 or 2, wherein, in the formula (1-1), R¹ is an n-propyl group.

5. The therapeutic agent for FTLD according to claim 3, wherein, in the formula (1-1), R¹ is an n-propyl group.

6. The therapeutic agent for FTLD according to claim 1 or 2, wherein the compound represented by the formula (1-1) is a compound represented by the following formula (1-2): 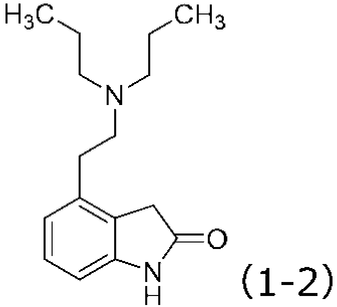 (4-[2-(dipropylamino)ethyl]-1,3-dihydro-2H-indol-2-one).

7. The therapeutic agent for FTLD according to claim 1 or 2, wherein the pharmaceutically acceptable salt of the compound represented by the formula (1-1) is the hydrochloride salt of the compound represented by the following formula (1-2): 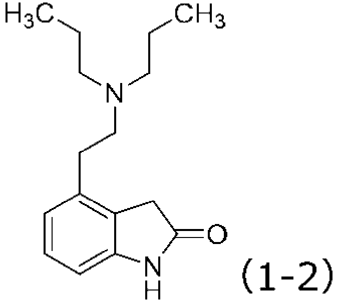 (4-[2-(dipropylamino)ethyl]-1,3-dihydro-2H-indol-2-one hydrochloride).

8. The therapeutic agent for FTLD according to claim 1 or 2, wherein the compound represented by the formula (2-1) is a compound represented by the following formula (2-2): 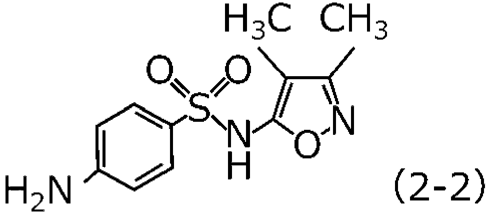 (4-amino-N-(3,4-dimethyl-5-isoxazoyl)benzenesulfonamide).

9. The therapeutic agent for FTLD according to claim 1 or 2, wherein the compound represented by the formula (3-1) is a compound represented by the following formula (3-2): 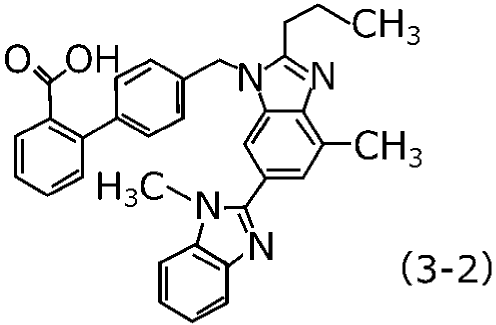 (4'-[[4-methyl-6-(1-methyl-1H-benzimidazol-2-yl)-2-propyl 1H-benzimidazol-1-yl]methyl]biphenyl-2-carboxylic acid).

10. A pharmaceutical composition for treating frontotemporal lobar degeneration (FTLD), comprising, as an active ingredient, a compound represented by the following formula (1-1): 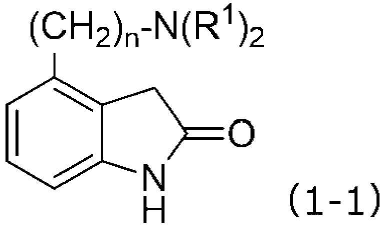
[in the formula (1-1), R¹ each independently represents an alkyl group having 1 to 6 carbon atoms or a 4-hydroxyphenethyl group, and n represents an integer from 1 to 3], a compound represented by the following formula (2-1): 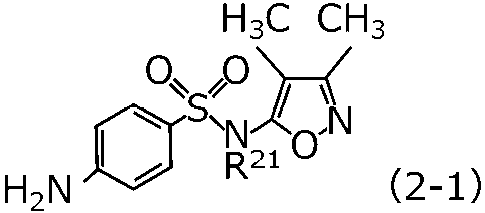
[in the formula (2-1), R²¹ is selected from the group consisting of hydrogen, fatty acid acyl groups having 2 to 18 carbon atoms, and aromatic carboxylic acid acyl groups having 7 to 9 carbon atoms],
or a compound represented by the following formula (3-1): 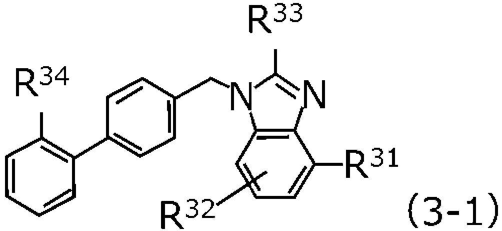
[in the formula (3-1), R³¹ is selected from the group consisting of hydrogen, halogen, alkyl groups having 1 to 6 carbon atoms, and CF₃;
R³² is selected from the group consisting of alkoxy groups having 1 to 6 carbon atoms substituted with an imidazolyl group, and optionally substituted nitrogen-containing aromatic heterocycles;
R³³ selected from the group consisting of hydrogen, alkyl groups having 1 to 6 carbon atoms, and cycloalkyl groups having 3 to 6 carbon atoms;
R³⁴ selected from the group consisting of carboxy groups, cyano groups, and 1H-tetrazolyl groups],
a pharmaceutically acceptable salt thereof, or a solvate thereof.
